# EUROPEAN PATENT APPLICATION

(11) **EP 4 847 513 A1**
(43) Date of publication of application: **02.12.2026**
(21) Application number: 25744572.6
(22) Date of filing: 21.01.2025
(51) Int. Cl.: C07D 498/22, A61K 31/529, A61P 35/00

(54) **CRYSTAL FORM OF MACROCYCLIC DERIVATIVE, PREPARATION METHOD THEREFOR AND USE THEREOF**

(30) Priority: 24.01.2024 CN 202410096274
(71) Applicant: Zhejiang Hisun Pharmaceutical Co., Ltd., Taizhou, Zhejiang 318000 (CN)
(72) Inventor: CHEN, Lianwei, Zhejiang 318000 (CN); LI, Na, Zhejiang 318000 (CN); SHI, Zhenjuan, Zhejiang 318000 (CN)
(74) Representative: Dehns
(86) International application number: PCT/CN2025/073615
(87) International publication number: WO 2025/157131

(57) **Abstract**

The present disclosure belongs to the field of chemical pharmaceuticals, relates to a crystal form of (3R,11R)-6-fluoro-3,11-dimethyl-10-oxa-2,13,17,18,21-pentaazapentacyclo[13.5.2.1^{8,11}.0^{4,9}.0^{18,22}]tric osan-1(21),4,6,8,15(22),16,19-heptaen-14-one, and a preparation method therefor and use thereof, and particularly to a crystal form C of a compound as shown in formula I and a preparation method therefor, and solves the problems of the compound as shown in formula I, such as a small particle size, poor flowability, poor stability and hygroscopicity.

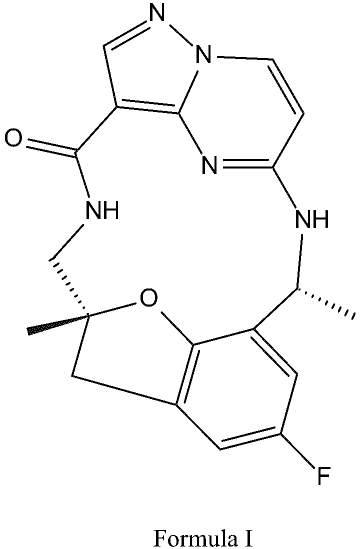

## Description

### TECHNICAL FIELD

The present disclosure relates to the field of chemical pharmaceuticals, and particularly to novel crystal forms A, C, D, E, F and G of a macrocyclic derivative (3R,11R)-6-fluoro-3,11-dimethyl-10-oxa-2,13,17,18,21-pentaazapentacyclo[13.5.2.1^{8,11}.0^{4,9}.0^{18,22}]tric osan-1(21),4,6,8,15(22),16,19-heptaen-14-one, and preparation methods therefor.

### BACKGROUND

In November, 2018, the U.S. FDA approved the listing of a TRK inhibitor Larotrectinib, which was a new oral drug for treating patients with abnormal TRK alterations. Previous studies have shown that the NTRK gene encoding TRK may fuse abnormally with other genes, resulting in cancer growing in many parts of the body, and the Larotrectinib can selectively inhibit TRK. Meanwhile, there are other TRK kinase inhibitors under study, including LOXO-195 (Loxo Oncology Inc, phase 2) and repotectinib (TP therapeutics Inc, phase 2). At present, a series of patent applications for TRK inhibitors have been published, including WO2015089139A1, WO2006082392A1, WO2007123269A1, etc. Although certain progress has been made in the research and application of the TRK inhibitors, there is still a huge room for improvement. Therefore, it is still necessary to continue to study and develop new TRK inhibitors.

WO2020233645A1 discloses a macrocyclic derivative having a structure as shown in formula I below, with a molecular formula C₁₉H₁₈FN₅O₂ and a chemical name: (3R,11R)-6-fluoro-3,11-dimethyl-10-oxa-2,13,17,18,21-pentaazapentacyclo[13.5.2.1^{8,11}.0^{4,9}.0^{18,22}]tric osan-1(21),4,6,8,15(22),16,19-heptaen-14-one. WO2020233645A1 further discloses a preparation method for the compound as shown in formula I and use thereof as an inhibitor of one or more protein kinases selected from TRK, ALK and ROS1, which indicates that the compound has good inhibitory activity against one or more protein kinases selected from TRK, ALK and ROS1.

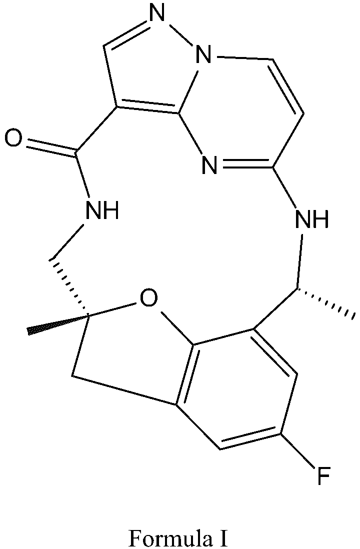

However, a solid of the compound as shown in formula I above prepared by the preparation method therefor disclosed in WO2020233645A1 is still unsatisfactory in terms of stability, particle size, flowability, hygroscopicity and the like.

At present, there is no report disclosing other crystal forms of the compound as shown in formula I in the prior art. Through a large number of experiments, the present inventors have obtained other crystal forms of the compound as shown in formula I.

### SUMMARY

In view of the problems in the prior art, the present disclosure provides crystal forms of a compound as shown in formula I, (3R,11R)-6-fluoro-3,11-dimethyl-10-oxa-2,13,17,18,21-pentaazapentacyclo[13.5.2.1^{8,11}.0^{4,9}.0^{18,22}]tric osan-1(21),4,6,8,15(22),16,19-heptaen-14-one, with a structure below, and preparation methods therefor.

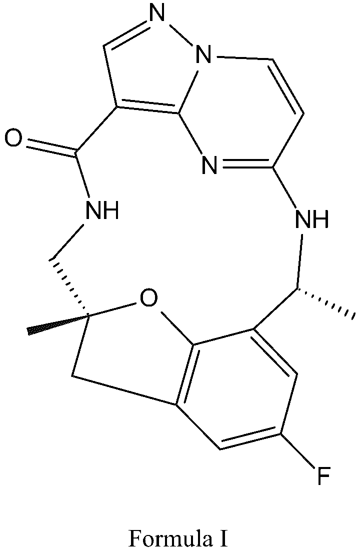

In a first aspect, the present disclosure provides a crystal form C of a compound as shown in formula I ((hereinafter referred to as the "crystal form C").

An X-ray powder diffraction pattern of the crystal form C using Cu-Kα radiation exhibits characteristic peaks at the following 2θ positions (expressed in degrees): 8.2+0.2°, 9.8+0.2°, 11.7±0.2°, 13.3+0.2°, 17.0±0.2°, 17.4±0.2°, 19.7+0.2°, 20.0+0.2°, 20.6+0.2°, 22.9+0.2° and 23.9+0.2°.

Preferably, an X-ray powder diffraction pattern of the crystal form C using Cu-Kα radiation exhibits characteristic peaks at the following 2θ positions (expressed in degrees): 8.2+0.2°, 9.8+0.2°, 11.7±0.2°, 12.5±0.2°, 13.3±0.2°, 14.3±0.2°, 15.7±0.2°, 15.9±0.2°, 16.5±0.2°, 17.0±0.2°, 17.4±0.2°, 17.8±0.2°, 18.0±0.2°, 19.4±0.2°, 19.7±0.2°, 20.0±0.2°, 20.6±0.2°, 21.5±0.2°, 21.9±0.2°, 22.9±0.2°, 23.9±0.2°, 24.6±0.2°, 25.7±0.2°, 27.3±0.2°, 28.7±0.2°, 29.5±0.2° and 30.3±0.2°.

More preferably, an X-ray powder diffraction pattern of the crystal form C using Cu-Kα radiation exhibits characteristic peaks with the corresponding relative intensities at the following 2θ positions (expressed in degrees):

**Table 1**

| Peak number | 2θ (°) | d (Å) | Relative intensity (%) |
|---|---|---|---|
| 1 | 8.2 | 10.74 | 100.0 |
| 2 | 9.8 | 9.01 | 61.8 |
| 3 | 10.6 | 8.32 | 0.6 |
| 4 | 11.7 | 7.51 | 20.9 |
| 5 | 12.5 | 7.04 | 3.9 |
| 6 | 13.3 | 6.65 | 15.3 |
| 7 | 14.3 | 6.16 | 5.0 |
| 8 | 15.1 | 5.83 | 0.7 |
| 9 | 15.7 | 5.63 | 4.1 |
| 10 | 15.9 | 5.53 | 3.6 |
| 11 | 16.5 | 5.34 | 1.9 |
| 12 | 17.0 | 5.18 | 14.5 |
| 13 | 17.4 | 5.08 | 58.6 |
| 14 | 17.8 | 4.97 | 7.1 |
| 15 | 18.0 | 4.90 | 12.3 |
| 16 | 18.8 | 4.70 | 1.8 |
| 17 | 19.4 | 4.55 | 10.6 |
| 18 | 19.7 | 4.48 | 47.4 |
| 19 | 20.0 | 4.43 | 40.0 |
| 20 | 20.6 | 4.29 | 33.8 |
| 21 | 21.5 | 4.12 | 5.1 |
| 22 | 21.9 | 4.04 | 5.1 |
| 23 | 22.9 | 3.87 | 22.4 |
| 24 | 23.9 | 3.71 | 30.8 |
| 25 | 24.6 | 3.61 | 13.0 |
| 26 | 24.9 | 3.57 | 4.6 |
| 27 | 25.7 | 3.45 | 6.8 |
| 28 | 26.4 | 3.36 | 5.8 |
| 29 | 27.0 | 3.29 | 10.1 |
| 30 | 27.3 | 3.25 | 14.0 |
| 31 | 27.9 | 3.19 | 4.7 |
| 32 | 28.7 | 3.09 | 6.3 |
| 33 | 29.5 | 3.01 | 7.5 |
| 34 | 29.9 | 2.982 | 5.0 |
| 35 | 30.3 | 2.94 | 8.4 |

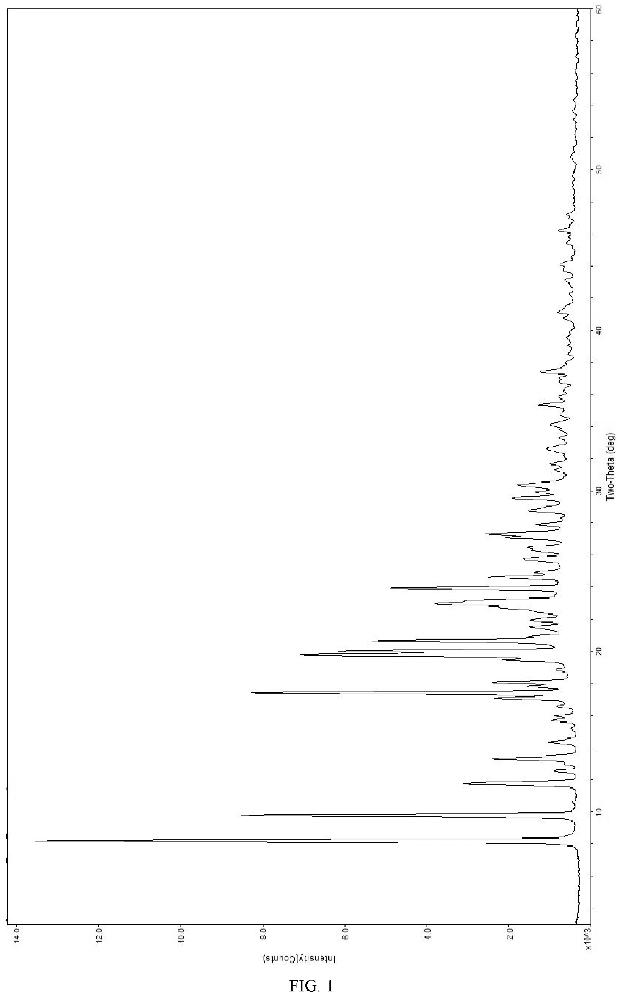

More preferably, the X-ray powder diffraction pattern of the crystal form C is basically as shown in FIG. 1.

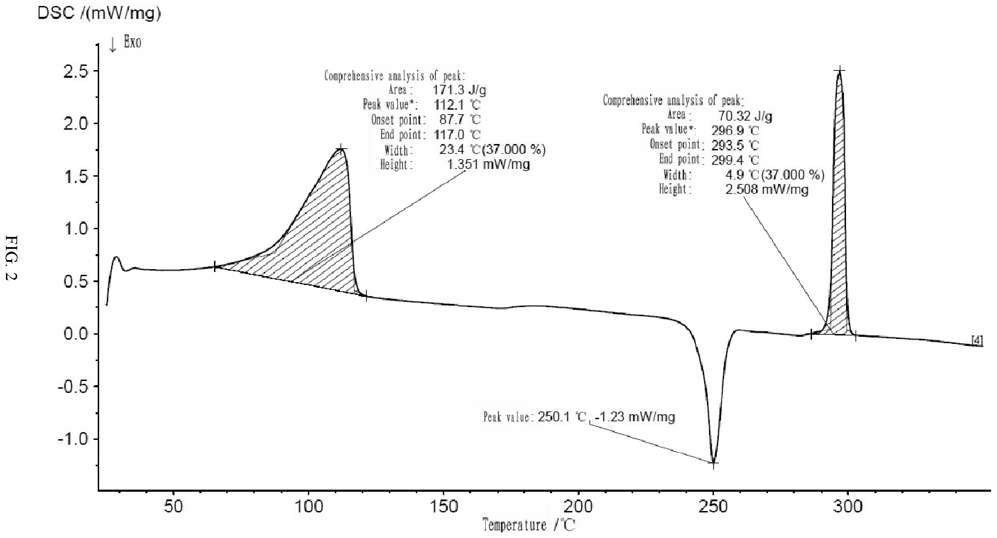

Further, a differential scanning calorimetry (DSC) pattern of the crystal form C exhibits endothermic peaks at 87-117°C and 293-299°C, and an inverted peak at 250°C, and the DSC thermogram is basically as shown in FIG. 2.

Still further, the crystal form C is a dihydrate, and a measured water content thereof is 8.95%.

Accordingly, the present disclosure provides a preparation method for the crystal form C, wherein the method comprises:
dissolving the compound as shown in formula I in a solvent to obtain a solution, wherein the solvent is methanol, ethanol, n-propanol, isopropanol, acetone, N,N-dimethylformamide, N,N-dimethylacetamide, dimethyl sulfoxide or a mixture thereof, adding water or adding the above solution into water to precipitate a solid, separating the solid, and drying in vacuum to obtain the crystal form C.

Preferably, a weight-to-volume ratio of the compound as shown in formula I to the solvent is 1: 10-1: 20, and a weight-to-volume ratio of the compound as shown in formula I to the water is 1: 20-1: 80, with a unit being g/mL.

Preferably, the drying in vacuum is carried out at a temperature of 20-60°C and lasts for 8-24 hours.

In another aspect, the present disclosure provides a pharmaceutical composition comprising a therapeutically effective amount of the crystal form C serving as an active ingredient. Preferably, in the pharmaceutical composition, the crystal form C may be mixed with one or more pharmaceutically acceptable solid or liquid diluents and/or excipients, and prepared into a Galenic formulation.

In yet another aspect, the present disclosure provides use of the crystal form C or the pharmaceutical composition in the manufacture of a medicament for treating a disease mediated by one or more protein kinases selected from TRK, ALK and ROS1, wherein, preferably, the disease mediated by one or more protein kinases selected from TRK, ALK and ROS1 is pain, cancer, inflammation, neurodegenerative disease or trypanosome infection, and more preferably, the cancer is neurocytoma, ovarian cancer, breast cancer, prostate cancer, gastric cancer, gastrointestinal tumor, liver cancer, cholangiocarcinoma, pancreatic cancer, multiple myeloma, astrocytoma, medulloblastoma, glioma, melanoma, thyroid cancer, lung cancer, large cell neuroendocrine tumor, colorectal cancer, secretory breast carcinoma, sarcoma, head and neck tumor and renal cancer.

In yet another aspect, the present disclosure provides use of the crystal form C or the pharmaceutical composition in the manufacture of an inhibitor of one or more protein kinases selected from TRK, ALK and ROS1.

In addition, the present disclosure further provides a method for treating a disease mediated by one or more protein kinases selected from TRK, ALK and ROS1, wherein the method comprises administering a therapeutically effective amount of the crystal form C to a subject in need thereof, wherein the disease mediated by one or more protein kinases selected from TRK, ALK and ROS1 is preferably pain, cancer, inflammation, neurodegenerative disease or trypanosome infection, wherein the cancer is preferably neurocytoma, ovarian cancer, breast cancer, prostate cancer, gastric cancer, gastrointestinal tumor, liver cancer, cholangiocarcinoma, pancreatic cancer, multiple myeloma, astrocytoma, medulloblastoma, glioma, melanoma, thyroid cancer, lung cancer, large cell neuroendocrine tumor, colorectal cancer, secretory breast carcinoma, sarcoma, head and neck tumor and renal cancer.

The present disclosure further provides a method for inhibiting one or more protein kinases selected from TRK, ALK and ROS1, wherein the method comprises contacting the crystal form C or the pharmaceutical composition comprising the crystal form C with one or more protein kinases selected from TRK, ALK and ROS1.

In another aspect, the present disclosure provides crystal form A of the compound as shown in formula I (hereinafter referred to as the "crystal form A").

An X-ray powder diffraction pattern of the crystal form A using Cu-Kα radiation exhibits characteristic peaks at the following 2θ positions (expressed in degrees): 6.7±0.2°, 7.7±0.2°, 10.4±0.2°, 13.4±0.2°, 16.5±0.2°, 19.7±0.2°, 20.2±0.2°, 21.4±0.2° and 23.3±0.2°.

Preferably, an X-ray powder diffraction pattern of the crystal form A using Cu-Kα radiation exhibits characteristic peaks at the following 2θ positions (expressed in degrees): 6.7±0.2°, 7.7±0.2°, 8.2±0.2°, 9.8±0.2°, 10.4±0.2°, 11.8±0.2°, 13.0±0.2°, 13.4±0.2°, 14.1±0.2°, 15.1±0.2°, 15.4±0.2°, 16.5±0.2°, 17.5±0.2°, 18.5±0.2°, 18.8±0.2°, 19.7±0.2°, 20.2±0.2°, 21.4±0.2°, 23.3±0.2°, 24.7±0.2°, 25.4±0.2° and 32.4±0.2°.

More preferably, an X-ray powder diffraction pattern of the crystal form A using Cu-Kα radiation exhibits characteristic peaks with corresponding relative intensities at the following 2θ positions (expressed in degrees):

**Table 2**

| Peak number | 2θ (°) | d (Å) | Relative intensity (%) |
|---|---|---|---|
| 1 | 6.7 | 13.10 | 85.6 |
| 2 | 7.7 | 11.44 | 100.0 |
| 3 | 8.2 | 10.67 | 7.0 |
| 4 | 9.8 | 8.94 | 5.1 |
| 5 | 10.4 | 8.44 | 34.3 |
| 6 | 11.8 | 7.49 | 2.3 |
| 7 | 13.0 | 6.79 | 4.5 |
| 8 | 13.4 | 6.58 | 11.3 |
| 9 | 14.1 | 6.23 | 5.5 |
| 10 | 15.1 | 5.84 | 2.1 |
| 11 | 15.4 | 5.71 | 3.2 |
| 12 | 16.5 | 5.36 | 10.5 |
| 13 | 17.5 | 5.05 | 3.4 |
| 14 | 18.5 | 4.78 | 5.6 |
| 15 | 18.8 | 4.71 | 6.3 |
| 16 | 19.7 | 4.49 | 13.4 |
| 17 | 20.2 | 4.37 | 12.7 |
| 18 | 21.1 | 4.19 | 9.7 |
| 19 | 21.4 | 4.14 | 17.7 |
| 20 | 21.9 | 4.04 | 6.7 |
| 21 | 22.4 | 3.95 | 3.3 |
| 22 | 22.9 | 3.87 | 3.4 |
| 23 | 23.3 | 3.80 | 36.3 |
| 24 | 24.7 | 3.59 | 7 |
| 25 | 25.4 | 3.49 | 8.8 |
| 26 | 25.8 | 3.43 | 3.7 |
| 27 | 26.3 | 3.38 | 4.4 |
| 28 | 26.8 | 3.31 | 4.2 |
| 29 | 27.2 | 3.26 | 7.6 |
| 30 | 27.9 | 3.19 | 2.9 |
| 31 | 28.6 | 3.11 | 0.7 |
| 32 | 29.1 | 3.06 | 2.9 |
| 33 | 29.6 | 3.01 | 3.5 |
| 34 | 30.3 | 2.93 | 2.1 |
| 35 | 31.3 | 2.85 | 3.6 |
| 36 | 31.7 | 2.81 | 1.6 |
| 37 | 32.4 | 2.75 | 8 |
| 38 | 33.5 | 2.67 | 3.1 |

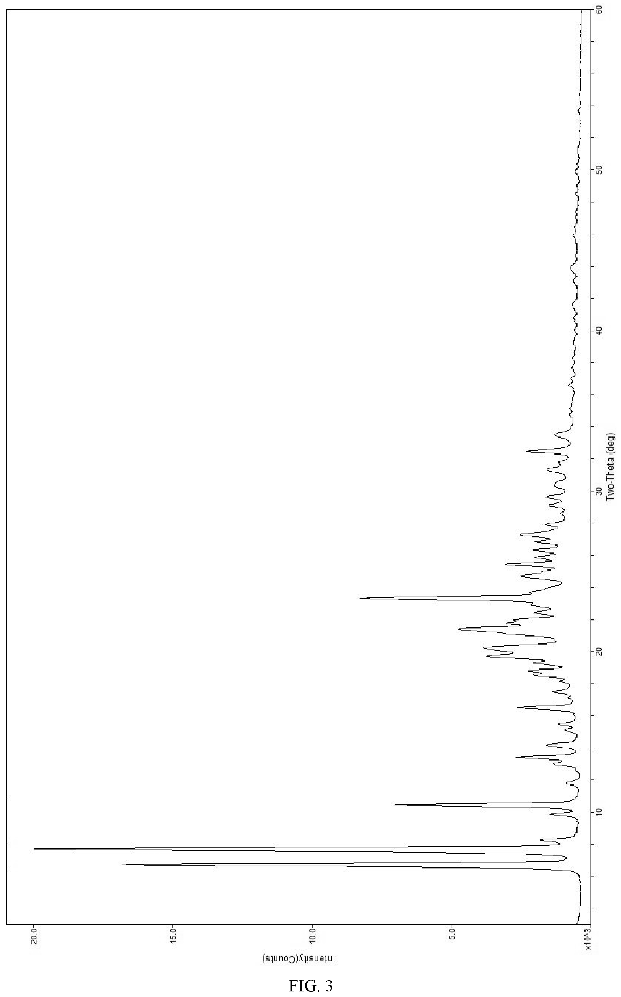

More preferably, the X-ray powder diffraction pattern of the crystal form A is basically as shown in FIG. 3.

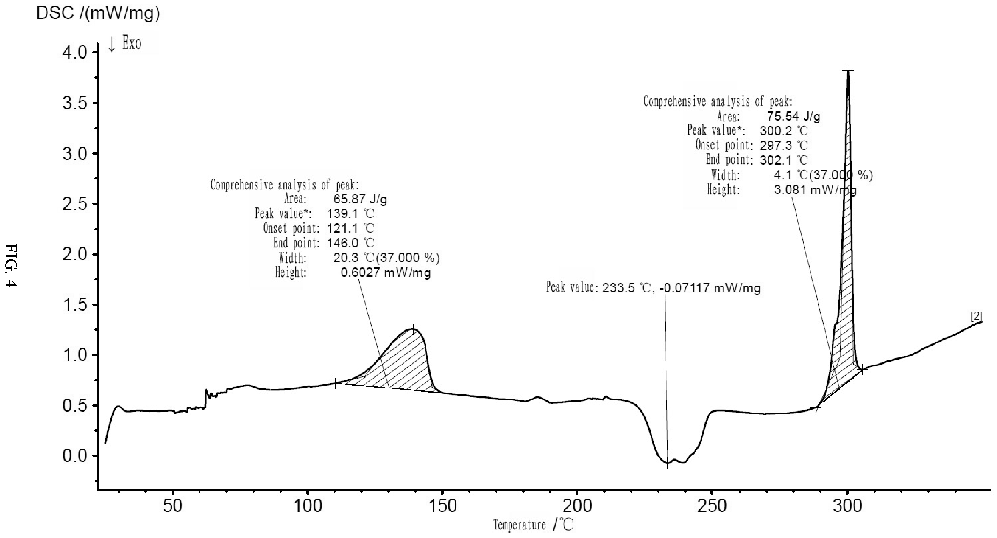

Further, a differential scanning calorimetry (DSC) pattern of the crystal form A exhibits endothermic peaks at 121-146°C and 297-302°C, and an inverted peak at 233°C, and the DSC thermogram is basically as shown in FIG. 4.

Accordingly, the present disclosure provides a preparation method for the crystal form A, wherein the method comprises:
adding the compound as shown in formula I into dichloromethane to be dissolved until the solution is clear, evaporating the solvent, separating the solid, and drying in vacuum to obtain the crystal form A.

Preferably, a weight-to-volume ratio of the compound as shown in formula I to the dichloromethane is 1: 50-1: 100, with a unit being g/mL.

Preferably, the drying in vacuum is carried out at a temperature of 20-60°C and lasts for 8-24 hours.

In another aspect, the present disclosure provides a crystal form D of the compound as shown in formula I (hereinafter referred to as the "crystal form D").

An X-ray powder diffraction pattern of the crystal form D using Cu-Kα radiation exhibits characteristic peaks at the following 2θ positions (expressed in degrees): 9.3±0.2°, 10.6±0.2°, 12.3±0.2°, 13.3±0.2°, 15.7±0.2°, 16.9±0.2°, 19.4±0.2°, 21.1±0.2°, 22.8±0.2° and 26.9±0.2°.

Preferably, an X-ray powder diffraction pattern of the crystal form D using Cu-Kα radiation exhibits characteristic peaks at the following 2θ positions (expressed in degrees): 9.3±0.2°, 10.6±0.2°, 12.3±0.2°, 13.3±0.2°, 15.7±0.2°, 16.9±0.2°, 19.4±0.2°, 21.1±0.2°, 22.8±0.2°, 24.2±0.2°, 24.9±0.2°, 25.5±0.2°, 26.9±0.2°, 27.9±0.2°, 32.3±0.2° and 33.4±0.2°.

More preferably, an X-ray powder diffraction pattern of the crystal form D using Cu-Kα radiation exhibits characteristic peaks with corresponding relative intensities at the following 2θ positions (expressed in degrees):

**Table 3**

| Peak number | 2θ (°) | d (Å) | Relative intensity (%) |
|---|---|---|---|
| 1 | 9.3 | 9.44 | 11.9 |
| 2 | 10.6 | 8.32 | 6.8 |
| 3 | 12.3 | 7.15 | 8.6 |
| 4 | 13.3 | 6.63 | 30.8 |
| 5 | 15.7 | 5.62 | 10.4 |
| 6 | 16.1 | 5.48 | 0.3 |
| 7 | 16.9 | 5.23 | 34 |
| 8 | 19.4 | 4.57 | 12.5 |
| 9 | 20.7 | 4.27 | 6.4 |
| 10 | 21.1 | 4.19 | 22.3 |
| 11 | 22.8 | 3.88 | 100 |
| 12 | 24.2 | 3.67 | 4.8 |
| 13 | 24.9 | 3.56 | 2.6 |
| 14 | 25.5 | 3.47 | 13.1 |
| 15 | 26.4 | 3.36 | 1.4 |
| 16 | 26.9 | 3.30 | 22.5 |
| 17 | 27.9 | 3.19 | 8.6 |
| 18 | 29.2 | 3.04 | 0.4 |
| 19 | 29.6 | 3.01 | 0.4 |
| 20 | 30.5 | 2.92 | 0.4 |
| 21 | 31.1 | 2.86 | 2.9 |
| 22 | 32.3 | 2.76 | 7.3 |
| 23 | 32.7 | 2.73 | 2.3 |
| 24 | 33.4 | 2.68 | 7.9 |
| 25 | 33.8 | 2.64 | 2.2 |
| 26 | 34.6 | 2.58 | 2.3 |
| 27 | 35.6 | 2.51 | 1.9 |
| 28 | 36.3 | 2.46 | 2.6 |
| 29 | 36.9 | 2.43 | 1.9 |

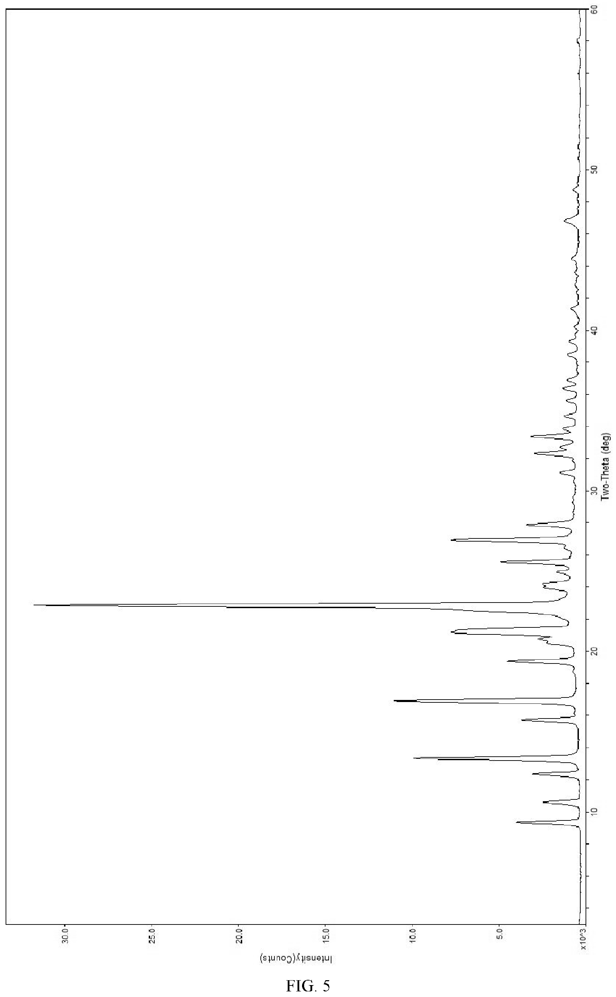

More preferably, the X-ray powder diffraction pattern of the crystal form D is basically as shown in FIG. 5.

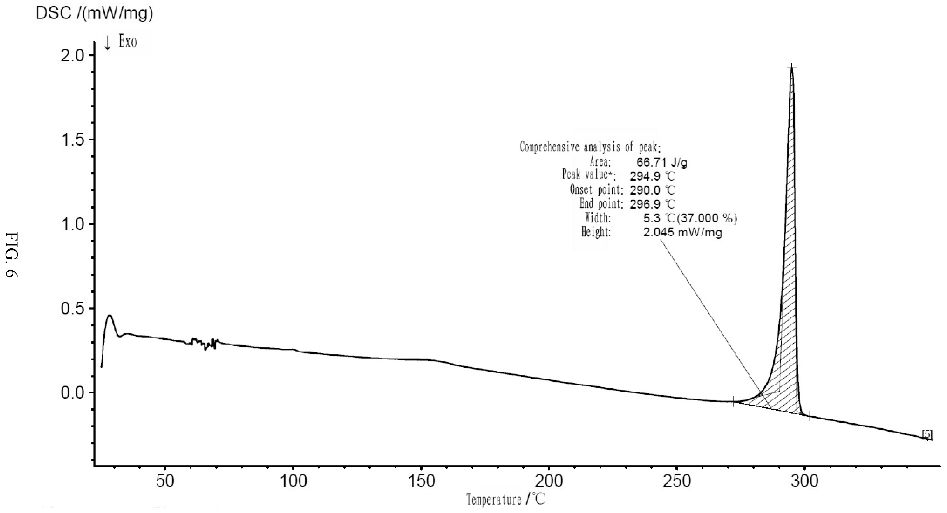

Further, a differential scanning calorimetry (DSC) pattern of the crystal form D exhibits an endothermic peak at 290-296°C, and the DSC thermogram is basically as shown in FIG. 6.

Accordingly, the present disclosure provides a preparation method for the crystal form D, wherein the method comprises:
dissolving the compound as shown in formula I in a solvent, wherein the solvent is ethanol, n-propanol, isopropanol, acetone, 2-butanone or a mixture thereof, adding n-heptane or adding the above solution into the n-heptane to precipitate a solid, separating the solid, and drying in vacuum to obtain the crystal form D.

Preferably, a weight-to-volume ratio of the compound as shown in formula I to the solvent is 1: 10-1: 20, and a weight-to-volume ratio of the compound as shown in formula I to the n-heptane is 1: 30-1: 80, with a unit being g/mL.

Preferably, the drying in vacuum is carried out at a temperature of 20-60°C and lasts for 8-24 hours.

In another aspect, the present disclosure provides a crystal form E of the compound as shown in formula I (hereinafter referred to as the "crystal form E").

An X-ray powder diffraction pattern of the crystal form E using Cu-Kα radiation exhibits characteristic peaks at the following 2θ positions (expressed in degrees): 8.1±0.2°, 9.2±0.2°, 9.7±0.2°, 11.7±0.2°, 12.2±0.2°, 13.2±0.2°, 15.6±0.2°, 16.8±0.2°, 17.4±0.2°, 19.3±0.2°, 20.0±0.2°, 21.0±0.2°, 22.8±0.2° and 26.8±0.2°.

Preferably, an X-ray powder diffraction pattern of the crystal form E using Cu-Kα radiation exhibits characteristic peaks at the following 2θ positions (expressed in degrees): 8.1±0.2°, 9.2±0.2°, 9.7±0.2°, 10.5±0.2°, 11.7±0.2°, 12.2±0.2°, 13.2±0.2°, 14.4±0.2°, 15.6±0.2°, 16.8±0.2°, 17.4±0.2°, 18.1±0.2°, 19.3±0.2°, 19.7±0.2°, 20.0±0.2°, 20.6±0.2°, 21.0±0.2°, 22.8±0.2°, 23.8±0.2°, 24.8±0.2°, 25.5±0.2°, 26.8±0.2°, 27.8±0.2° and 33.3±0.2°.

More preferably, an X-ray powder diffraction pattern of the crystal form E using Cu-Kα radiation exhibits characteristic peaks with corresponding relative intensities at the following 2θ positions (expressed in degrees):

**Table 4**

| Peak number | 2θ (°) | d (Å) | Relative intensity (%) |
|---|---|---|---|
| 1 | 8.1 | 10.79 | 27.2 |
| 2 | 9.2 | 9.52 | 33.9 |
| 3 | 9.7 | 9.03 | 29.6 |
| 4 | 10.5 | 8.40 | 5.0 |
| 5 | 11.7 | 7.51 | 14.5 |
| 6 | 12.2 | 7.19 | 26.7 |
| 7 | 13.2 | 6.67 | 40.9 |
| 8 | 14.4 | 6.14 | 2.0 |
| 9 | 15.6 | 5.65 | 19.6 |
| 10 | 16.0 | 5.52 | 3.3 |
| 11 | 16.8 | 5.25 | 49.1 |
| 12 | 17.4 | 5.08 | 18.2 |
| 13 | 18.1 | 4.89 | 8.6 |
| 14 | 19.3 | 4.58 | 26.1 |
| 15 | 19.7 | 4.49 | 16.8 |
| 16 | 20.0 | 4.42 | 22.8 |
| 17 | 20.6 | 4.29 | 17.7 |
| 18 | 21.0 | 4.21 | 38.5 |
| 19 | 22.4 | 3.95 | 25.0 |
| 20 | 22.8 | 3.89 | 100 |
| 21 | 23.1 | 3.83 | 13.7 |
| 22 | 23.8 | 3.72 | 15.0 |
| 23 | 24.8 | 3.57 | 8.5 |
| 24 | 25.5 | 3.49 | 13.0 |
| 25 | 26.4 | 3.37 | 5.8 |
| 26 | 26.8 | 3.31 | 28.5 |
| 27 | 27.3 | 3.25 | 4.3 |
| 28 | 27.8 | 3.20 | 12.8 |
| 29 | 28.7 | 3.10 | 2.4 |
| 30 | 29.6 | 3.01 | 6.2 |
| 31 | 30.2 | 2.94 | 4.7 |
| 32 | 31.0 | 2.87 | 3.6 |
| 33 | 31.7 | 2.81 | 1.9 |
| 34 | 32.2 | 2.77 | 6.6 |
| 35 | 32.6 | 2.74 | 5.6 |
| 36 | 33.3 | 2.68 | 8.3 |

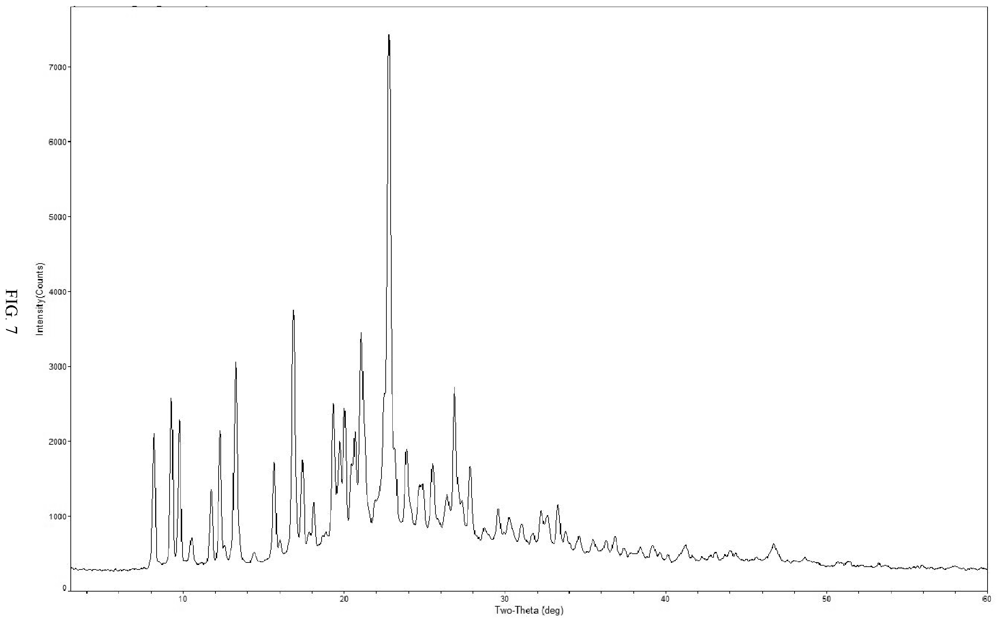

More preferably, the X-ray powder diffraction pattern of the crystal form E is basically as shown in FIG. 7.

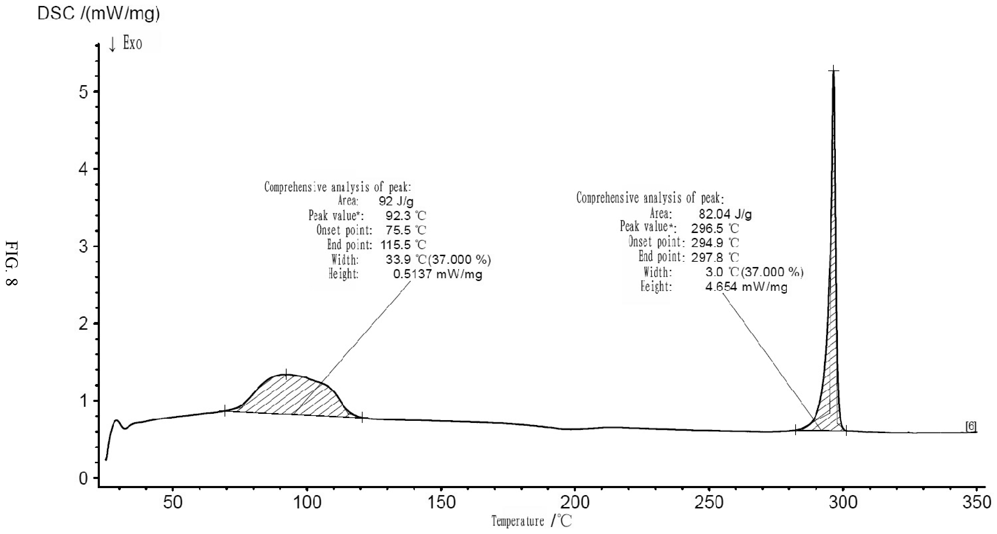

Further, a differential scanning calorimetry (DSC) pattern of the crystal form E exhibits endothermic peaks at 75-115°C and 294-297°C, and the DSC thermogram is basically as shown in FIG. 8.

Accordingly, the present disclosure provides a preparation method for the crystal form E, wherein the method comprises:
adding the compound as shown in formula I into acetone, stirring the mixture at room temperature to be dissolved until the solution is clear, evaporating the solvent naturally, separating the solid, and drying in vacuum to obtain the crystal form E.

Preferably, a weight-to-volume ratio of the compound as shown in formula I to the acetone is 1: 10-1: 20, with a unit being g/mL.

Preferably, the drying in vacuum is carried out at a temperature of 20-60°C and lasts for 8-24 hours.

In another aspect, the present disclosure provides a crystal form F of the compound as shown in formula I (hereinafter referred to as the "crystal form F").

An X-ray powder diffraction pattern of the crystal form F using Cu-Kα radiation exhibits characteristic peaks at the following 2θ positions (expressed in degrees): 6.5±0.2°, 9.7±0.2°, 10.8±0.2°, 12.1±0.2°, 17.5±0.2°, 18.6±0.2°, 19.9±0.2°, 21.3±0.2°, 23.3±0.2°, 23.7±0.2°, 24.8±0.2° and 29.1±0.2°.

Preferably, an X-ray powder diffraction pattern of the crystal form F using Cu-Kα radiation exhibits characteristic peaks at the following 2θ positions (expressed in degrees): 6.5±0.2°, 9.0±0.2°, 9.7±0.2°, 10.3±0.2°, 10.8±0.2°, 11.5±0.2°, 12.1±0.2°, 13.6±0.2°, 14.1±0.2°, 14.6±0.2°, 15.5±0.2°, 17.1±0.2°, 17.5±0.2°, 18.1±0.2°, 18.6±0.2°, 19.9±0.2°, 20.3±0.2', 21.0±0.2°, 21.3±0.2°, 22.8±0.2°, 23.3±0.2°, 23.7±0.2°, 24.5±0.2°, 24.8±0.2°, 27.6±0.2°, 29.1±0.2° and 30.0±0.2°.

More preferably, an X-ray powder diffraction pattern of the crystal form F using Cu-Kα radiation exhibits characteristic peaks with corresponding relative intensities at the following 2θ positions (expressed in degrees):

**Table 5**

| Peak number | 2θ (°) | d (Å) | Relative intensity (%) |
|---|---|---|---|
| 1 | 6.5 | 13.50 | 31.6 |
| 2 | 9.0 | 9.75 | 16.1 |
| 3 | 9.7 | 9.07 | 59.7 |
| 4 | 10.3 | 8.50 | 11.2 |
| 5 | 10.8 | 8.17 | 37.5 |
| 6 | 11.5 | 7.63 | 5.2 |
| 7 | 12.1 | 7.27 | 45.6 |
| 8 | 13.1 | 6.70 | 2.1 |
| 9 | 13.6 | 6.46 | 9.1 |
| 10 | 14.1 | 6.24 | 14.4 |
| 11 | 14.6 | 6.05 | 16.4 |
| 12 | 15.5 | 5.71 | 16 |
| 13 | 16.1 | 5.47 | 2.4 |
| 14 | 17.1 | 5.17 | 9 |
| 15 | 17.5 | 5.05 | 52.2 |
| 16 | 18.1 | 4.88 | 11.6 |
| 17 | 18.6 | 4.76 | 40.6 |
| 18 | 18.9 | 4.67 | 9.1 |
| 19 | 19.6 | 4.51 | 21.3 |
| 20 | 19.9 | 4.45 | 66.9 |
| 21 | 20.3 | 4.35 | 11.3 |
| 22 | 20.7 | 4.28 | 4.6 |
| 23 | 21.0 | 4.22 | 12.6 |
| 24 | 21.3 | 4.15 | 32.5 |
| 25 | 22.2 | 3.99 | 2.2 |
| 26 | 22.5 | 3.93 | 5.3 |
| 27 | 22.8 | 3.88 | 14.8 |
| 28 | 23.3 | 3.80 | 87.5 |
| 29 | 23.7 | 3.74 | 100 |
| 30 | 24.5 | 3.62 | 22.7 |
| 31 | 24.8 | 3.58 | 26.9 |
| 32 | 25.1 | 3.53 | 8 |
| 33 | 25.8 | 3.44 | 11.9 |
| 34 | 26.1 | 3.40 | 7.1 |
| 35 | 26.9 | 3.30 | 2.7 |
| 36 | 27.6 | 3.21 | 20.3 |
| 37 | 28.7 | 3.10 | 1.2 |
| 38 | 29.1 | 3.06 | 24.8 |
| 39 | 29.8 | 2.99 | 13.1 |
| 40 | 30.0 | 2.96 | 14.3 |
| 41 | 30.7 | 2.90 | 5.8 |
| 42 | 31.0 | 2.87 | 3.3 |
| 43 | 32.7 | 2.73 | 5.8 |
| 44 | 33.6 | 2.65 | 4.3 |
| 45 | 34.6 | 2.58 | 2.4 |
| 46 | 35.2 | 2.54 | 4.6 |
| 47 | 37.5 | 2.39 | 1.9 |
| 48 | 38.3 | 2.34 | 6.1 |
| 49 | 39.5 | 2.27 | 6.1 |

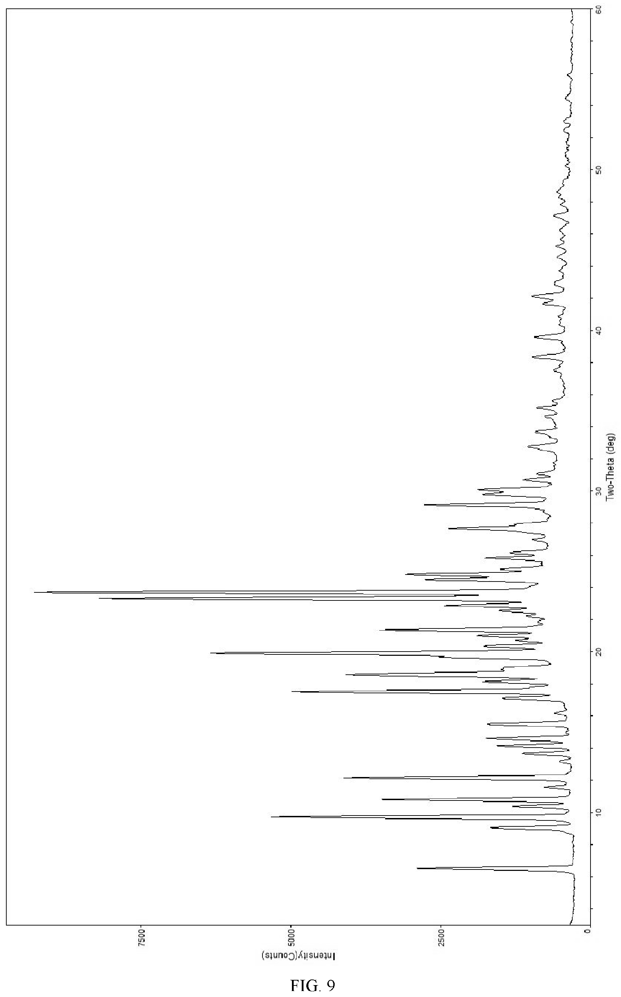

More preferably, the X-ray powder diffraction pattern of the crystal form F is basically as shown in FIG. 9.

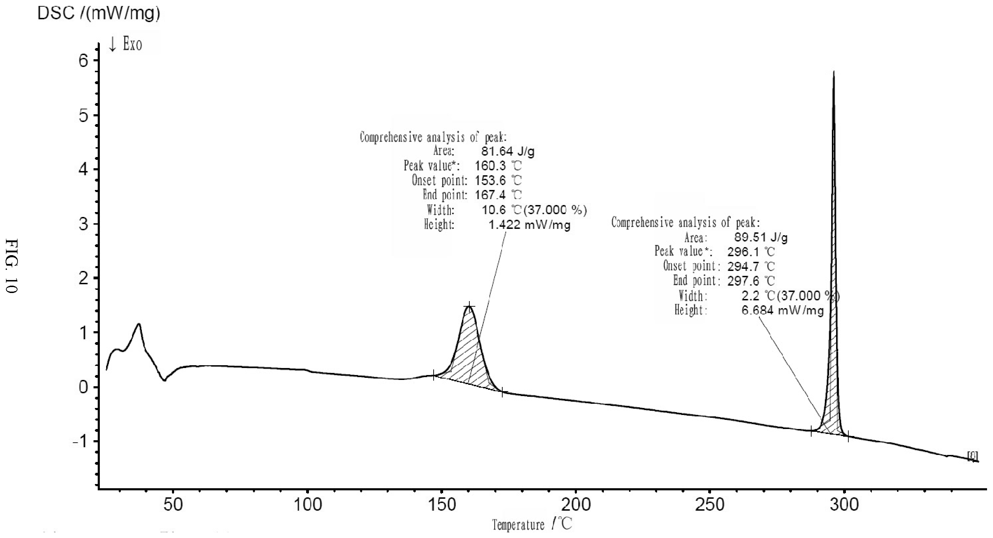

Further, a differential scanning calorimetry (DSC) pattern of the crystal form F exhibits an endothermic peak at 153-167°C and an endothermic peak at 294-297°C, and the DSC thermogram is basically as shown in FIG. 10.

Accordingly, the present disclosure provides a preparation method for the crystal form F, wherein the method comprises:
adding the crystal form B of the compound as shown in formula I into methyl isobutyl ketone, suspending and stirring the mixture at 20-40°C for 2-24 hours, then separating the solid, and drying in vacuum to obtain the crystal form F.

Preferably, a weight-to-volume ratio of the compound as shown in formula I to the methyl isobutyl ketone is 1: 10-1: 20, with a unit being g/mL.

Preferably, the drying in vacuum is carried out at a temperature of 20-60°C and lasts for 8-24 hours.

In another aspect, the present disclosure provides a crystal form G of the compound as shown in formula I (hereinafter referred to as the "crystal form G").

An X-ray powder diffraction pattern of the crystal form G using Cu-Kα radiation exhibits characteristic peaks at the following 2θ positions (expressed in degrees): 6.5±0.2°, 8.2±0.2°, 9.8±0.2°, 11.0±0.2°, 17.4±0.2°, 19.0±0.2°, 20.0±0.2°, 21.6±0.2°, 23.8±0.2°, 28.0±0.2° and 30.2±0.2°.

Preferably, an X-ray powder diffraction pattern of the crystal form G using Cu-Kα radiation exhibits characteristic peaks at the following 2θ positions (expressed in degrees): 6.5±0.2°, 8.2±0.2°, 9.1±0.2°, 9.8±0.2°, 10.5±0.2°, 11.0±0.2°, 11.7±0.2°, 12.3±0.2°, 13.8±0.2°, 14.3±0.2°, 15.7±0.2°, 17.4±0.2°, 17.7±0.2°, 18.1±0.2°, 18.6±0.2°, 19.0±0.2°, 19.7±0.2°, 20.0±0.2°, 21.6±0.2°, 23.1±0.2°, 23.8±0.2°, 24.1±0.2°, 25.0±0.2°, 26.2±0.2°, 28.0±0.2° and 30.2±0.2°.

More preferably, an X-ray powder diffraction pattern of the crystal form G using Cu-Kα radiation exhibits characteristic peaks with corresponding relative intensities at the following 2θ positions (expressed in degrees):

**Table 5**

| Peak number | 2θ (°) | d (Å) | Relative intensity (%) |
|---|---|---|---|
| 1 | 6.5 | 13.38 | 24.3 |
| 2 | 8.2 | 10.77 | 36.3 |
| 3 | 9.1 | 9.62 | 16.8 |
| 4 | 9.8 | 8.96 | 87.9 |
| 5 | 10.5 | 8.35 | 14.4 |
| 6 | 11.0 | 7.99 | 32.8 |
| 7 | 11.7 | 7.55 | 25.2 |
| 8 | 12.3 | 7.15 | 24.4 |
| 9 | 13.5 | 6.55 | 6.6 |
| 10 | 13.8 | 6.39 | 14.6 |
| 11 | 14.3 | 6.17 | 20.9 |
| 12 | 14.8 | 5.94 | 7.9 |
| 13 | 15.7 | 5.63 | 14.1 |
| 14 | 15.9 | 5.53 | 4.4 |
| 15 | 17.0 | 5.19 | 6.5 |
| 16 | 17.4 | 5.08 | 38.4 |
| 17 | 17.7 | 4.97 | 30.7 |
| 18 | 18.1 | 4.89 | 19.1 |
| 19 | 18.6 | 4.74 | 15.4 |
| 20 | 19.0 | 4.66 | 46.3 |
| 21 | 19.4 | 4.55 | 6.6 |
| 22 | 19.7 | 4.48 | 26.0 |
| 23 | 20.0 | 4.41 | 84.0 |
| 24 | 20.7 | 4.28 | 10.3 |
| 25 | 21.0 | 4.22 | 4.6 |
| 26 | 21.3 | 4.15 | 12.8 |
| 27 | 21.6 | 4.09 | 27.6 |
| 28 | 22.3 | 3.97 | 2.2 |
| 29 | 23.1 | 3.83 | 19.6 |
| 30 | 23.8 | 3.72 | 100.0 |
| 31 | 24.1 | 3.67 | 18.2 |
| 32 | 24.6 | 3.60 | 13.8 |
| 33 | 25.0 | 3.55 | 21.3 |
| 34 | 25.6 | 3.47 | 6.5 |
| 35 | 26.2 | 3.39 | 14.3 |
| 36 | 27.1 | 3.28 | 9.5 |
| 37 | 27.3 | 3.25 | 11.5 |
| 38 | 28.0 | 3.17 | 34.5 |
| 39 | 29.0 | 3.07 | 3.6 |
| 40 | 29.3 | 3.04 | 9.6 |
| 41 | 29.6 | 3.01 | 11.3 |
| 42 | 30.2 | 2.94 | 20.1 |
| 43 | 31.3 | 2.85 | 3.4 |
| 44 | 31.6 | 2.82 | 4.4 |
| 45 | 32.4 | 2.75 | 4.3 |

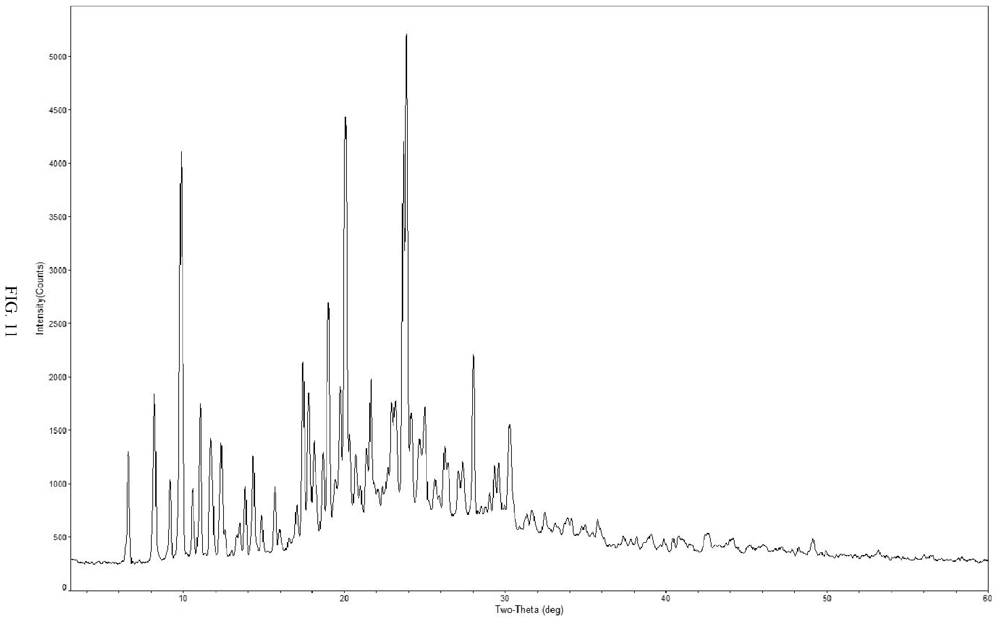

More preferably, the X-ray powder diffraction pattern of the crystal form G is basically as shown in FIG. 11.

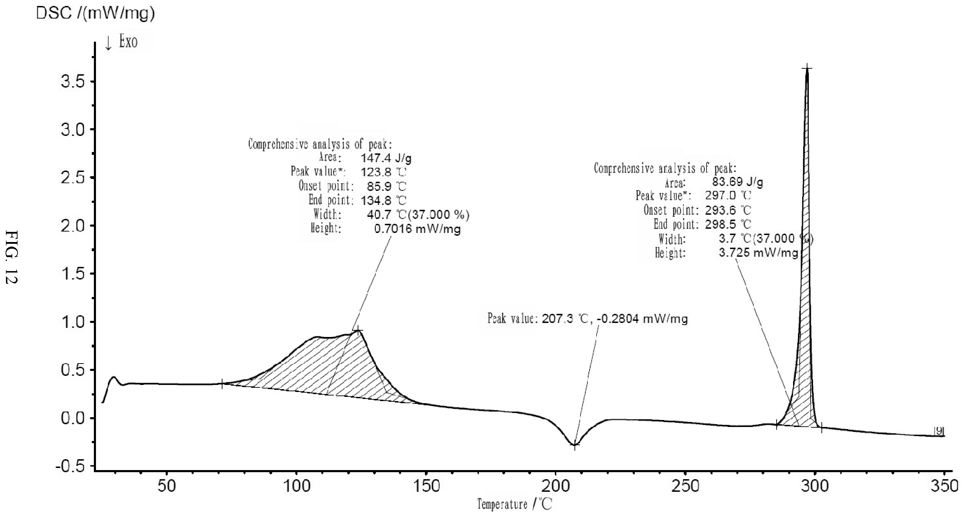

Further, a differential scanning calorimetry (DSC) pattern of the crystal form G exhibits endothermic peaks at 85-134°C and 293-298°C, and an inverted peak at 207°C, and the DSC thermogram is basically as shown in FIG. 12.

Accordingly, the present disclosure provides a preparation method for the crystal form G, wherein the method comprises:
adding the compound as shown in formula I into 2-butanone, stirring the mixture at room temperature to be dissolved until the solution is clear, evaporating the solvent naturally, separating the solid, and drying in vacuum to obtain the crystal form G.

Preferably, a weight-to-volume ratio of the compound as shown in formula I to the 2-butanone is 1: 10-1: 20, with a unit being g/mL.

Preferably, the drying in vacuum is carried out at a temperature of 20-60°C and lasts for 8-24 hours.

In another aspect, the solid of the compound as shown in formula I obtained by the preparation method disclosed in WO2020233645A1 is a crystal form B (hereinafter referred to as the "crystal form B").

An X-ray powder diffraction pattern of the crystal form B using Cu-Kα radiation exhibits characteristic peaks at the following 2θ positions (expressed in degrees) 6.4±0.2°, 9.3±0.2°, 10.8±0.2°, 11.9±0.2°, 13.3±0.2°, 17.4±0.2°, 19.3±0.2°, 21.1±0.2°, 21.9±0.2°, 23.9±0.2° and 26.8±0.2°.

Preferably, an X-ray powder diffraction pattern of the crystal form B using Cu-Kα radiation exhibits characteristic peaks at the following 2θ positions (expressed in degrees) 6.4±0.2°, 7.2±0.2°, 9.3±0.2°, 10.0±0.2°, 10.8±0.2°, 11.9±0.2°, 13.3±0.2°, 14.8±0.2°, 16.2±0.2°, 17.4±0.2°, 18.5±0.2°, 19.3±0.2°, 20.0±0.2°, 21.1±0.2°, 21.9±0.2°, 23.2±0.2°, 23.9±0.2°, 25.6±0.2°, 26.8±0.2°, 27.8±0.2° and 28.8±0.2°.

More preferably, an X-ray powder diffraction pattern of the crystal form B using Cu-Kα radiation exhibits characteristic peaks with corresponding relative intensities at the following 2θ positions (expressed in degrees):

**Table 5**

| Peak number | 2θ (°) | d (Å) | Relative intensity (%) |
|---|---|---|---|
| 1 | 6.4 | 13.62 | 47.5 |
| 2 | 7.2 | 12.23 | 9.1 |
| 3 | 9.3 | 9.46 | 23.6 |
| 4 | 10.0 | 8.81 | 14.9 |
| 5 | 10.8 | 8.17 | 49.0 |
| 6 | 11.9 | 7.41 | 37.8 |
| 7 | 12.3 | 7.14 | 3.9 |
| 8 | 13.3 | 6.63 | 100.0 |
| 9 | 14.2 | 6.22 | 2.8 |
| 10 | 14.8 | 5.96 | 13.5 |
| 11 | 16.2 | 5.44 | 7.8 |
| 12 | 17.4 | 5.08 | 17.9 |
| 13 | 18.5 | 4.76 | 3.7 |
| 14 | 19.3 | 4.58 | 37.7 |
| 15 | 20.0 | 4.43 | 11.3 |
| 16 | 21.1 | 4.19 | 41.4 |
| 17 | 21.9 | 4.05 | 54.4 |
| 18 | 23.2 | 3.81 | 33.4 |
| 19 | 23.9 | 3.72 | 59.5 |
| 20 | 25.6 | 3.46 | 18.1 |
| 21 | 26.2 | 3.38 | 6.1 |
| 22 | 26.8 | 3.31 | 51.6 |
| 23 | 27.8 | 3.20 | 26.2 |
| 24 | 28.8 | 3.09 | 15.7 |
| 25 | 29.2 | 3.05 | 10.5 |
| 26 | 30.5 | 2.92 | 5.5 |
| 27 | 32.7 | 2.73 | 4.3 |

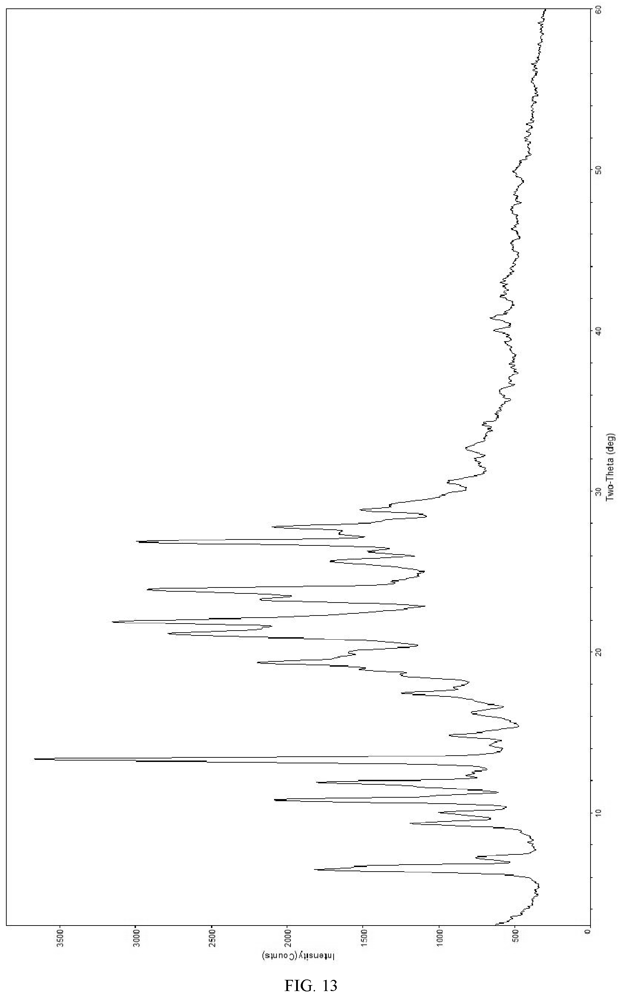

More preferably, the X-ray powder diffraction pattern of the crystal form B is basically as shown in FIG. 13.

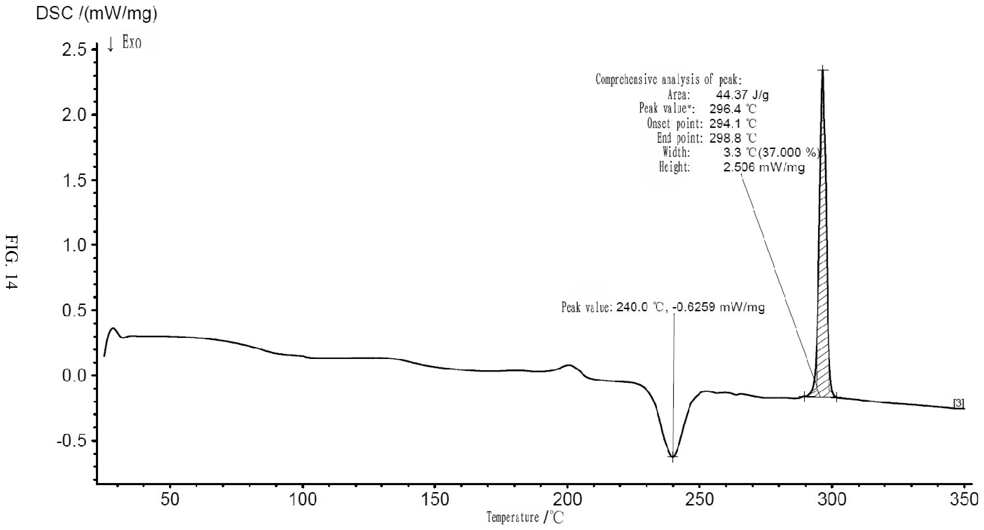

Further, a differential scanning calorimetry (DSC) pattern of the crystal form B exhibits an endothermic peak at 294-298°C, and an inverted peak at 240°C, and the DSC thermogram is basically as shown in FIG. 14.

Compared with the prior art, the beneficial technical effects achieved by the present disclosure primarily reside in:present disclosure:
a crystallization process of the crystal form C of the compound as shown in formula I of the present disclosure is simple, convenient to operate, low in pollution and capable of being industrialized, and in addition, the drug of the crystal form of the present disclosure simultaneously possesses the advantages of high product purity, excellent physicochemical properties, good high-temperature/high-humidity and chemical and physical stability, excellent processing (filtration, drying, dissolution and tableting) adaptability and reproducibility, and further has relatively large particle size, hygroscopicity and good flowability, which solves the problems of small particle size, poor flowability, poor stability and hygroscopicity in the compound as shown in formula I, thereby being conducive to the processing of drug and the improvement of drug formulation properties, and having a promising market application prospect.

### BRIEF DESCRIPTION OF THE DRAWINGS

FIG. 1 is an X-ray powder diffraction pattern of crystal form C prepared in Example 1.
FIG. 2 is a DSC thermogram of crystal form C prepared in Example 1.
FIG. 3 is an X-ray powder diffraction pattern of crystal form A prepared in Example 12.
FIG. 4 is a DSC thermogram of crystal form A prepared in Example 12.
FIG. 5 is an X-ray powder diffraction pattern of crystal form D prepared in Example 14.
FIG. 6 is a DSC thermogram of crystal form D prepared in Example 14.
FIG. 7 is an X-ray powder diffraction pattern of crystal form E prepared in Example 22.
FIG. 8 is a DSC thermogram of crystal form E prepared in Example 22.
FIG. 9 is an X-ray powder diffraction pattern of crystal form F prepared in Example 24.
FIG. 10 is a DSC thermogram of crystal form F prepared in Example 24.
FIG. 11 is an X-ray powder diffraction pattern of crystal form G prepared in Example 26.
FIG. 12 is a DSC thermogram of crystal form G prepared in Example 26.
FIG. 13 is an X-ray powder diffraction pattern of crystal form B prepared in a preparation example.
FIG. 14 is a DSC thermogram of crystal form B prepared in the preparation example.

### DETAILED DESCRIPTION

### General definitions and terms

Unless otherwise stated, all technical and scientific terms used herein have the same meanings as those commonly understood by those of ordinary skills in the art to which the present disclosure belongs. In case of any inconsistency, the definitions provided in the present application shall prevail. When a quantity, a concentration, or other value or parameter is expressed in terms of a range, a preferred range, or a preferred upper value limit and a preferred lower value limit, it should be understood as specifically disclosing any range formed by combining any upper limit or preferred value with any lower limit or preferred value of such range, without considering whether such range is specifically disclosed. Unless otherwise stated, numerical ranges set forth herein are intended to include endpoints of the range and all integers and fractions within the range.

The term "about" or "approximately", when used in conjunction with a numerical variable, generally refers to a value of the variable and all values of the variable within an experimental error (such as falling within a 95% confidence interval for a mean), or within ±10% of a specified value, or within a wider range.

The expression "comprise", or a synonymous expression such as "include", "contain" or "have", is open-ended, and does not exclude additional unrecited elements, steps or components. The expression "composed of ..." excludes any element, step or component not specified. The expression "basically composed of ..." refers to limiting a range to specified elements, steps or components, plus optional elements, steps or components that do not materially affect basic and novel characteristics of the claimed subject matter. It should be understood that the expression "comprise" encompasses the expressions "basically composed of ..." and "composed of ...".

The term "optional" or "optionally" used herein means that the subsequently described event or situation may or may not occur, and the description includes the case in which the event or situation occurs and the case in which the event or situation does not occur.

Unless otherwise stated, all percentages, parts and the like herein are by weight.

As used herein, the term "crystal form" or "crystal" refers to any solid substance exhibiting three-dimensional ordering, which, in contrast to an amorphous solid substance, gives a characteristic X-ray powder diffraction pattern with well-defined peaks.

As used herein, the term "amorphous" refers to any solid substance that is non-ordered in three dimensions.

As used herein, the term "X-ray powder diffraction (XRPD) pattern" refers to an experimentally observed diffraction pattern, or parameters, data or values derived therefrom. The XRPD pattern is typically characterized by peak positions (abscissa) and/or peak intensities (ordinate).

As used herein, the term "2θ" refers to a peak position expressed in degrees (°) based on the setup in an X-ray diffraction experiment, and is usually a unit of an abscissa in the diffraction pattern. If a reflection is diffracted when an incident beam forms an angle θ with a lattice plane, the experimental setup requires recording a reflected beam at an angle 2θ. It should be understood that a specific 2θ value mentioned herein for a particular crystal form is intended to denotes a 2θ value (expressed in degrees) measured by using X-ray diffraction experimental conditions described herein. For example, as described herein, Cu-Kα (Kα1 is 1.5418 Å) is used as a radiation source. The XRPD pattern herein may be collected, for example, on a Rigaku MiniFlex-600 X-ray powder diffractometer. Exemplary test conditions may be a scan speed of 10°/min and a scan step size of 0.01°.

As used herein, the term "basically" with respect to an X-ray diffraction peak means that variations in representative peak position and intensity are taken into account. For example, those skilled in the art may understand that the peak position (2θ) may exhibit some variations, typically up to 0.1-0.2° (±0.1° to ±0.2°), and the instrument used for diffraction measurement may also cause some variations. In addition, those skilled in the art may understand that the relative peak intensity may vary due to an instrumental difference, as well as a crystallinity, a preferred orientation, a surface of prepared sample and other factors known to those skilled in the art, which should be regarded as qualitative measurement only.

As used herein, a transition temperature at which a crystal absorbs or releases heat as a result of a change in crystal structure or crystal melting is determined by differential scanning calorimetry (DSC). For the same crystal form of the same compound, errors in a thermal transition temperature and a melting point are typically within about 5°C in successive analyses. When describing a compound as having a given DSC peak or melting point, it refers to such DSC peak or melting point ±5°C. The term "basically" also takes such temperature variation into account. The DSC provides an auxiliary method for distinguishing different crystal forms. Different crystal forms may be identified based on characteristic transition temperatures thereof. It is pointed out that, for a mixture, the DSC peak or melting point may vary within a wider range. Furthermore, because the melting of a substance is accompanied with decomposition, a melting temperature is related to a heating rate. The DSC thermogram may be measured, for example, on an instrument with a model such as NETZSCH DSC214 Polyma. Exemplary test conditions are a heating rate of 10°C/min and a temperature range of 25-300°C.

### Pharmaceutical composition and administration

In one embodiment, the present disclosure provides a pharmaceutical composition, and the composition comprises a crystal form of a compound as shown in formula I, and one or more pharmaceutically acceptable carriers.

As used herein, the term "pharmaceutically acceptable carrier" refers to a solid or liquid diluent, adjuvant, excipient or vehicle with which a therapeutic agent is administered, which, within a scope of sound medical judgment, is suitable for contact with human and/or other animal tissues without undue toxicity, irritation, allergic response, or other problems or complications commensurate with a reasonable benefit/risk ratio.

The pharmaceutically acceptable carriers that may be used in the pharmaceutical composition of the present disclosure comprise, but are not limited to, sterile liquids, such as water and oils, comprising those originates from petroleum, animal, vegetable or synthetic origin, for example, soybean oil, peanut oil and mineral oil. When the pharmaceutical composition is administered intravenously, the water is an exemplary carrier. Saline solutions, and aqueous dextrose and glycerol solutions may also be used as liquid carriers, particularly for injectable solutions. Suitable pharmaceutical excipients comprise dextrose, starch, lactose, gelatin, maltose, sucrose, chalk, silica gel, glyceryl monostearate, sodium stearate, talc, sodium chloride, glycerol, propylene glycol, water, ethanol, and the like. The composition may further comprise a minor amount of wetting agent, emulsifying agent or pH buffering agent as desired. An oral formulation may comprise a standard carrier, such as pharmaceutical-grade mannitol, lactose, starch, magnesium stearate, cellulose, sodium saccharin and magnesium carbonate. An example of the suitable pharmaceutically acceptable carrier is as described in Remington's Pharmaceutical Sciences (1990).

The composition of the present disclosure can act systemically and/or locally. For this purpose, they can be administered by a suitable route, such as injection, intraarterial, subcutaneous, intravenous, intraperitoneal, intramuscular or transdermal administration; or oral, nasal, sublingual, transmucosal or local administration, or administration in a form of ophthalmic preparation, or inhalation administration.

For these administration routes, the composition of the present disclosure may be administered in a suitable dosage form. The dosage form comprises, but is not limited to, a tablet, a pill, a capsule, a lozenge, a pastille, a powder, a spray, a cream, an ointment, a suppository, a gel, an aqueous suspension, an injection, an elixir and a syrup.

The pharmaceutical composition of the present disclosure may be prepared by any method well known in the art, such as mixing, dissolving, granulating, sugar-coating, grinding, emulsifying and lyophilizing. As used herein, the term "therapeutically effective amount" refers to an amount of a compound which, when administered, alleviates to some extent one or more symptoms of a disease treated.

A dosage regimen may be adjusted to provide an optimum response desired. For example, a single bolus may be administered, and several divided doses may be administered over time, or the dose may be proportionally reduced or increased as indicated by an exigency of a therapeutic situation. It should be noted that a dose value may vary with the type and severity of a condition to be alleviated, and may comprise single or multiple doses. It should be further understood that, for any particular subject, a specific dosage regimen should be adjusted over time according to a need of the subject and a professional judgment of a person administering the composition or supervising administration thereof.

Unless otherwise indicated, as used herein, the term "treatment" means reversing, alleviating or inhibiting a disease or condition to which such term applies or the progression of one or more symptoms of such disease or condition, or preventing such disease or condition or the one or more symptoms of such condition or disorder.

As used herein, the "subject" comprises a human or non-human animal subject. An exemplary human subject comprises a human subject (referred to as a patient) suffering from a disease (such as the disease described herein) or a normal subject. The "non-human animal" subject in the present disclosure comprises all vertebrates, for example, non-mammals (such as amphibians, reptiles and birds) and mammals, such as non-human primates, domestic animals, and/or farmed animals (such as dogs, cats, sheep, cattle and pigs).

The following examples further illustrate the present disclosure. However, it is necessary to point out that the following examples are only for the description of the summary and do not constitute any limitation on the scope of protection of the present disclosure, and the scope of protection of the present disclosure shall be defined by the claims.

Preparation and characterization of the crystal forms of the compound as shown in formula I

### Preparation Example

A compound as shown in formula I in the examples of the present disclosure was prepared by a method in Example 8 of Patent WO2020233645A1. Specifically, 5-(((1R)-1-((2R)-2-(aminomethyl)-5-fluoro-2-methyl-2,3-dihydrobenzofuran-7-yl)ethyl)amino)pyraz olo[1,5-a]pyrimidine-3-carboxylic acid 8h (797 mg), pentafluorophenyl diphenylphosphinate (1.08 g, 2.48 mmol) and N,N-diisopropylethylamine (2.14 g, 16.56 mmol) were dissolved in 12 mL of mixed solvent (dichloromethane: N,N-dimethylformamide = 5: 1), and the reaction was carried out at room temperature overnight. After completion of the reaction was monitored by LC-MS, 30 mL of saturated ammonium chloride solution was added, and the mixture was extracted with dichloromethane (30 mL × 3). The organic phases were combined, dried with anhydrous sodium sulfate, filtered, and concentrated under reduced pressure. The product was purified by preparative liquid chromatography (column: AKZONOBEL Kromasil; 250×21.2 mm I.D.; 5 µm, 20 mL/min; mobile phase A: 0.05%TFA+H₂O, and mobile phase B: CH₃CN) to obtain (3R,11R)-6-fluoro-3,11-dimethyl-10-oxa-2,13,17,18,21-pentaazapentacyclo[13.5.2.1^{8,11}.0^{4,9}.0^{18,22}]tric osan-1(21),4,6,8,15(22),16,19-heptaen-14-one (200 mg). The obtained solid was identified as a crystal form B by XRPD, and an X-ray powder diffraction pattern and a DSC thermogram of the crystal form were as shown in FIG.13 and FIG. 14 respectively.

### Test instrument information and method

An X-ray powder diffraction instrument and test conditions involved in the present disclosure were as follows: an X-ray diffraction instrument with a model Rigaku MiniFlex-600, Cu target; and an operating method: a scan rate of 10°/min and a scan step size of 0.01°.

DSC test conditions involved in the present disclosure were as follows: a DSC detector with a model NETZSCH DSC 214 Polyma; an operating method: a heating rate of 10°C/min and a temperature range of 25-350°C.

High-performance liquid chromatography (HPLC) test conditions involved in the present disclosure were as follows: a liquid chromatograph with a model Agilent 1260; a column of Waters XBridge C18 4.6*150 mm, 3.5 µm or an equivalent column; a detection wavelength of 226 nm; and a column temperature of 30°C.

### Example 1 Preparation of crystal form C

0.1 g of the compound as shown in formula I was dissolved in 1 mL of ethanol, and 2 mL of water was added dropwise. A solid was precipitated, separated and dried in vacuum at 50°C for 24 hours. The product was identified as crystal form C, and an XRPD pattern and a DSC thermogram of the crystal form were as shown in FIG. 1 and FIG. 2 respectively.

### Example 2 Preparation of crystal form C

0.1 g of the compound as shown in formula I was dissolved in 2 mL of ethanol, and 4 mL of water was added dropwise. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 3 Preparation of crystal form C

0.05 g of the compound as shown in formula I was dissolved in 1 mL of ethanol, and the solution was added dropwise into 4 mL of water. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 4 Preparation of crystal form C

0.05 g of the compound as shown in formula I was dissolved in 1 mL of methanol, and 2 mL of water was added dropwise. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 5 Preparation of crystal form C

0.05 g of the compound as shown in formula I was dissolved in 1 mL of n-propanol, and 4 mL of water was added dropwise. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 6 Preparation of crystal form C

0.05 g of the compound as shown in formula I was dissolved in 1 mL of isopropanol, and 4 mL of water was added dropwise. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 7 Preparation of crystal form C

0.05 g of the compound as shown in formula I was dissolved in 1 mL of acetone, and 4 mL of water was added dropwise. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 8 Preparation of crystal form C

0.05 g of the compound as shown in formula I was dissolved in 1 mL of N,N-dimethylformamide, and 4 mL of water was added dropwise. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 9 Preparation of crystal form C

0.05 g of the compound as shown in formula I was dissolved in 1 mL of N,N-dimethylacetamide, and 4 mL of water was added dropwise. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 10 Preparation of crystal form C

0.05 g of the compound as shown in formula I was dissolved in 1 mL of dimethyl sulfoxide, and 2 mL of water was added dropwise. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 11 Preparation of crystal form C

0.05 g of the compound as shown in formula I was dissolved in a mixed solvent of 0.5 mL of methanol and 0.5 mL of ethanol, and 4 mL of water was added dropwise. A solid was precipitated, separated and dried in vacuum at 50°C for 24 hours. The product was identified as crystal form C, and an XRPD pattern and a DSC thermogram of the crystal form were consistent with those shown in FIG. 1 and FIG. 2 respectively.

### Example 12 Preparation of crystal form A

0.1 g of the compound as shown in formula I was dissolved in 5 mL of dichloromethane. The solvent was evaporated. A solid was precipitated, separated and dried in vacuum at 50°C for 24 hours. The product was identified as crystal form A, and an XRPD pattern and a DSC thermogram of the crystal form were as shown in FIG. 3 and FIG. 4 respectively.

### Example 13 Preparation of crystal form A

0.1 g of the compound as shown in formula I was dissolved in 10 mL of dichloromethane. The solvent was evaporated. A solid was precipitated, separated and dried in vacuum at 50°C for 24 hours. The product was identified as crystal form A, and an XRPD pattern and a DSC thermogram of the crystal form were consistent with those shown in FIG. 3 and FIG. 4 respectively.

### Example 14 Preparation of crystal form D

0.1 g of the compound as shown in formula I was dissolved in 1 mL of ethanol, and 3 mL of n-heptane was added dropwise. A solid was precipitated, separated and dried in vacuum at 50°C for 24 hours. The product was identified as crystal form D, and an XRPD pattern and a DSC thermogram of the crystal form were as shown in FIG. 5 and FIG. 6 respectively.

### Example 15 Preparation of crystal form D

0.05 g of the compound as shown in formula I was dissolved in 1 mL of ethanol, and 4 mL of n-heptane was added dropwise. A solid was precipitated, separated and dried in vacuum at 50°C for 24 hours. The product was identified as crystal form D, and an XRPD pattern and a DSC thermogram of the crystal form were consistent with those shown in FIG. 5 and FIG. 6 respectively.

### Example 16 Preparation of crystal form D

0.05 g of the compound as shown in formula I was dissolved in 1 mL of ethanol, and the solution was added dropwise into 4 mL of n-heptane. A solid was precipitated, separated and dried in vacuum at 50°C for 24 hours. The product was identified as crystal form D, and an XRPD pattern and a DSC thermogram of the crystal form were consistent with those shown in FIG. 5 and FIG. 6 respectively.

### Example 17 Preparation of crystal form D

0.05 g of the compound as shown in formula I was dissolved in 1 mL of n-propanol, and 4 mL of n-heptane was added dropwise. A solid was precipitated, separated and dried in vacuum at 50°C for 24 hours. The product was identified as crystal form D, and an XRPD pattern and a DSC thermogram of the crystal form were consistent with those shown in FIG. 5 and FIG. 6 respectively.

### Example 18 Preparation of crystal form D

0.05 g of the compound as shown in formula I was dissolved in 1 mL of isopropanol, and 4 mL of n-heptane was added dropwise. A solid was precipitated, separated and dried in vacuum at 50°C for 24 hours. The product was identified as crystal form D, and an XRPD pattern and a DSC thermogram of the crystal form were consistent with those shown in FIG. 5 and FIG. 6 respectively.

### Example 19 Preparation of crystal form D

0.05 g of the compound as shown in formula I was dissolved in 1 mL of acetone, and 4 mL of n-heptane was added dropwise. A solid was precipitated, separated and dried in vacuum at 50°C for 24 hours. The product was identified as crystal form D, and an XRPD pattern and a DSC thermogram of the crystal form were consistent with those shown in FIG. 5 and FIG. 6 respectively.

### Example 20 Preparation of crystal form D

0.05 g of the compound as shown in formula I was dissolved in 1 mL of 2-butanone, and 4 mL of n-heptane was added dropwise. A solid was precipitated, separated and dried in vacuum at 50°C for 24 hours. The product was identified as crystal form D, and an XRPD pattern and a DSC thermogram of the crystal form were consistent with those shown in FIG. 5 and FIG. 6 respectively.

### Example 21 Preparation of crystal form D

0.05 g of the compound as shown in formula I was dissolved in a mixed solvent of 0.5 mL of ethanol and 0.5 mL of acetone, and 4 mL of n-heptane was added dropwise. A solid was precipitated, separated and dried in vacuum at 50°C for 24 hours. The product was identified as crystal form D, and an XRPD pattern and a DSC thermogram of the crystal form were consistent with those shown in FIG. 5 and FIG. 6 respectively.

### Example 22 Preparation of crystal form E

0.05 g of the compound as shown in formula I was dissolved in 0.5 mL of acetone. The solvent was evaporated. A solid was precipitated, separated and dried in vacuum at 50°C for 24 hours. The product was identified as crystal form E, and an XRPD pattern and a DSC thermogram of the crystal form were as shown in FIG. 7 and FIG. 8 respectively.

### Example 23 Preparation of crystal form E

0.05 g of the compound as shown in formula I was dissolved in 1 mL of acetone. The solvent was evaporated. A solid was precipitated, separated and dried in vacuum at 50°C for 24 hours. The product was identified as crystal form E, and an XRPD pattern and a DSC thermogram of the crystal form were consistent with those shown in FIG. 7 and FIG. 8 respectively.

### Example 24 Preparation of crystal form F

0.1 g of crystal form B of the compound as shown in formula I was added into 1 mL of methyl isobutyl ketone. The mixture was suspended and stirred at 20°C for 24 hours. A solid was separated and dried in vacuum at 50°C for 24 hours. The product was identified as crystal form F, and an XRPD pattern and a DSC thermogram of the crystal form were as shown in FIG. 9 and FIG. 10 respectively.

### Example 25 Preparation of crystal form F

0.05 g of crystal form B of the compound as shown in formula I was added into 1 mL of methyl isobutyl ketone. The mixture was suspended and stirred at 40°C for 2 hours. A solid was separated and dried in vacuum at 50°C for 24 hours. The product was identified as crystal form F, and an XRPD pattern and a DSC thermogram of the crystal form were consistent with those shown in FIG. 9 and FIG. 10 respectively.

### Example 26 Preparation of crystal form G

0.05 g of the compound as shown in formula I was dissolved in 0.5 mL of 2-butanone. The solvent was evaporated. A solid was precipitated, separated and dried in vacuum at 50°C for 24 hours. The product was identified as crystal form G, and an XRPD pattern and a DSC thermogram of the crystal form were as shown in FIG. 11 and FIG. 12 respectively.

### Example 27 Preparation of crystal form G

0.05 g of the compound as shown in formula I was dissolved in 1 mL of 2-butanone. The solvent was evaporated. A solid was precipitated, separated and dried in vacuum at 50°C for 24 hours. The product was identified as crystal form G, and an XRPD pattern and a DSC thermogram of the crystal form were consistent with those shown in FIG. 11 and FIG. 12 respectively.

### Preparation of crystal form B of the compound as shown in formula I

The crystal form B of the compound as shown in formula I was prepared according to Example 8 of Patent WO2020233645A1.

### Stability test

The crystal form B of the compound as shown in formula I prepared in the preparation example, the crystal form C of the compound as shown in formula I prepared in Example 1, the crystal form A of the compound as shown in formula I prepared in Example 12, the crystal form D of the compound as shown in formula I prepared in Example 14, the crystal form E of the compound as shown in formula I prepared in Example 22, the crystal form F of the compound as shown in formula I prepared in Example 24 and the crystal form G of the compound as shown in formula I prepared in Example 26 were placed under 75% RH at 40°C for 6 months and under 60% RH at 25°C for 12 months respectively. Contents of the crystal forms were determined. Specific results can be seen in Table 6.

**Table 6**

| | Crystal form A | Crystal form B | Crystal form C | Crystal form D | Crystal form E | Crystal form F | Crystal form G |
|---|---|---|---|---|---|---|---|
| 0 day | 97.8% | 97.8% | 98.6% | 98.5% | 98.1% | 98.3% | 97.9% |
| Under 75%RH at 40°C for 6 months | 97.2% | 97.4% | 98.6% | 98.3% | 97.6% | 98.0% | 97.3% |
| Under 60%RH at 25°C for 12 months | 97.4% | 97.5% | 98.6% | 98.4% | 97.7% | 98.1% | 97.5% |

It could be seen from the results in Table 6 that, compared with the crystal form B, the crystal form C exhibited better stability and higher purity, and the crystal form C had superior stability relative to other crystal forms.

### Particle size test

The crystal form B of the compound as shown in formula I prepared in the preparation example, the crystal form C of the compound as shown in formula I prepared in Example 1, the crystal form A of the compound as shown in formula I prepared in Example 12, the crystal form D of the compound as shown in formula I prepared in Example 14, the crystal form E of the compound as shown in formula I prepared in Example 22, the crystal form F of the compound as shown in formula I prepared in Example 24 and the crystal form G of the compound as shown in formula I prepared in Example 26 were subjected to particle size tests respectively. Specific results can be seen in Table 7.

**Table 7**

| | Crystal form A | Crystal form B | Crystal form C | Crystal form D | Crystal form E | Crystal form F | Crystal form G |
|---|---|---|---|---|---|---|---|
| D50 | 13.52 µm | 28.62 µm | 182.53 µm | 52.58 µm | 23.64 µm | 35.36 µm | 26.64 µm |
| D90 | 38.45 µm | 55.11 µm | 534.77 µm | 108.47 µm | 59.56 µm | 68.43 µm | 65.56 µm |

It could be seen from the results in Table 7 that the crystal form B had a significantly smaller particle size than the crystal form C (wherein D50 was 28.62 µm, and not more than 16% of D50 of the crystal form of the present disclosure; D90 was 55.11 µm, and not more than 11% of D90 of the crystal form of the present disclosure), and the crystal form C had a significantly larger particle size than other crystal forms. Therefore, the present disclosure was more suitable for a forming process of a solid formulation (such as a tablet, a capsule or a granule).

### Flowability experiment

The crystal form B of the compound as shown in formula I prepared in the preparation example, the crystal form C of the compound as shown in formula I prepared in Example 1, the crystal form A of the compound as shown in formula I prepared in Example 12, the crystal form D of the compound as shown in formula I prepared in Example 14, the crystal form E of the compound as shown in formula I prepared in Example 22, the crystal form F of the compound as shown in formula I prepared in Example 24 and the crystal form G of the compound as shown in formula I prepared in Example 26 were subjected to repose angle tests respectively. Specific results can be seen in Table 8.

**Table 8**

| | Crystal form A | Crystal form B | Crystal form C | Crystal form D | Crystal form E | Crystal form F | Crystal form G |
|---|---|---|---|---|---|---|---|
| 1 | 47.6° | 46.5° | 31.4° | 42.6° | 45.8° | 44.8° | 45.6 |
| 2 | 47.4° | 46.9° | 31.1° | 42.8° | 45.9° | 44.5° | 45.2 |
| 3 | 47.6° | 46.2° | 31.2° | 42.5° | 46.1° | 44.7° | 44.9 |

The above results indicated that the repose angle of the crystal form B was significantly larger than that of the crystal form C, thereby demonstrating that the flowability of the crystal form C was obviously superior to that of the crystal form B, and the repose angle of the crystal form C was significantly smaller than those of other crystal forms. Therefore, the present disclosure was more suitable for a forming process of a solid formulation (such as a tablet, a capsule or a granule).

### Hygroscopicity experiment

The crystal form B of the compound as shown in formula I prepared in the preparation example, the crystal form C of the compound as shown in formula I prepared in Example 1, the crystal form A of the compound as shown in formula I prepared in Example 12, the crystal form D of the compound as shown in formula I prepared in Example 14, the crystal form E of the compound as shown in formula I prepared in Example 22, the crystal form F of the compound as shown in formula I prepared in Example 24 and the crystal form G of the compound as shown in formula I prepared in Example 26 were placed under 75%RH at 25°C for 24 hours, and weights of the crystal forms were measured for hygroscopicity calculation. Specific results can be seen in Table 9.

**Table 9**

| | Crystal form A | Crystal form B | Crystal form C | Crystal form D | Crystal form E | Crystal form F | Crystal form G |
|---|---|---|---|---|---|---|---|
| Hygroscopicity | 0.15% | 0.13% | 0.01% | 0.05% | 0.09% | 0.07% | 0.09 |

The above results indicated that the hygroscopicity of the crystal form B was significantly higher than that of the crystal form C, and the hygroscopicity of the crystal form C was significantly lower than those of other crystal forms, thereby demonstrating that the crystal form C was obviously superior to the crystal form B and other crystal forms in terms of hygroscopicity.

### Crystal transformation experiment

The crystal form B of the compound as shown in formula I prepared in the preparation example and the crystal form D of the compound as shown in formula I prepared in Example 14 were stirred in water at room temperature for 24 hours, and the mixture was filtered and dried, and then subjected to an XRD test. Specific results can be seen in Table 10.

**Table 10**

| Initial crystal form | Final crystal form |
|---|---|
| Crystal form B | Crystal form C |
| Crystal form D | Crystal form C |

The above results indicated that the crystal form B and the crystal form D were easily transformed into the crystal form C in water, thereby demonstrating that the crystal form C exhibited better stability than the crystal form B and the crystal form D in the presence of water.

Those skilled in the art can understand that the values or value endpoints involved in the technical solution of the present disclosure are not intended to be limited to the numbers themselves in terms of their meanings or intended scope of protection, and they contain the acceptable error ranges widely recognized in the art, such as experimental errors, measurement errors, statistical errors and random errors, all of which are included within the scope of the present disclosure.

Those skilled in the art will appreciate that many modifications and variations of the present disclosure may be made without departing from the spirit and scope thereof. The specific embodiments described herein are provided by way of example only and are not meant to be limiting in any way. The true scope and spirit of the present disclosure are indicated by the appended claims, and the specification and examples are illustrative only.

## Claims

1. A crystal form C of a compound as shown in formula I, 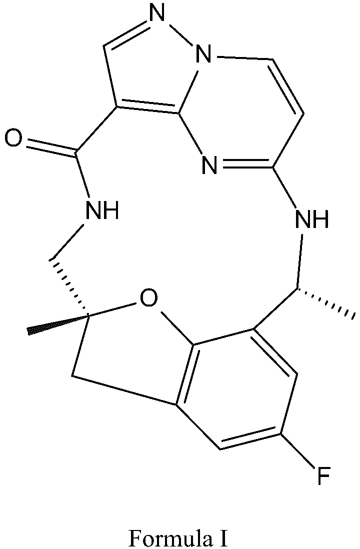 wherein, an X-ray powder diffraction pattern of the crystal form C using Cu-Kα radiation exhibits characteristic peaks at the following 2θ positions (expressed in degrees): 8.2±0.2°, 9.8+0.2°, 11.7±0.2°, 13.3±0.2°, 17.0±0.2°, 17.4±0.2°, 19.7±0.2°, 20.0±0.2° 20.6±0.2°, 22.9±0.2° and 23.9+0.2°.

2. The crystal form C according to claim 1, wherein the X-ray powder diffraction pattern of the crystal form C using Cu-Kα radiation exhibits characteristic peaks at the following 2θ positions (expressed in degrees): 8.2±0.2°, 9.8+0.2°, 11.7±0.2°, 12.5±0.2°, 13.3±0.2°, 14.3±0.2°, 15.7±0.2°, 15.9±0.2°, 16.5±0.2°, 17.0±0.2°, 17.4±0.2°, 17.8±0.2°, 18.0±0.2°, 19.4±0.2°, 19.7±0.2°, 20.0±0.2°, 20.6±0.2°, 21.5±0.2°, 21.9±0.2°, 22.9±0.2°, 23.9±0.2°, 24.6±0.2°, 25.7±0.2°, 27.3±0.2°, 28.7±0.2°, 29.5±0.2° and 30.3±0.2°.

3. The crystal form C according to claim 1 or 2, wherein the X-ray powder diffraction pattern of the crystal form C is as shown in FIG. 1.

4. The crystal form C according to any one of claims 1 to 3, wherein the crystal form C is a dihydrate.

5. A preparation method for the crystal form C according to any one of claims 1 to 4, wherein the method comprises:
dissolving the compound as shown in formula I in a solvent to obtain a solution, wherein the solvent is methanol, ethanol, n-propanol, isopropanol, acetone, N,N-dimethylformamide, N,N-dimethylacetamide, dimethyl sulfoxide or a mixture thereof, adding water or adding the above solution into water to precipitate a solid, separating the solid, and drying in vacuum to obtain the crystal form C.

6. The method according to claim 5, wherein a weight-to-volume ratio of the compound as shown in formula I to the solvent is 1: 10-1: 20, and a weight-to-volume ratio of the compound as shown in formula I to the water is 1: 20-1: 80, with a unit being g/mL.

7. A pharmaceutical composition, comprising a therapeutically effective amount of the crystal form C according to any one of claims 1 to 4 and a pharmaceutically acceptable excipient.

8. Use of the crystal form C according to any one of claims 1 to 4 or the pharmaceutical composition according to claim 7 in the manufacture of a medicament for treating a disease mediated by one or more protein kinases selected from TRK, ALK and ROS1, wherein, preferably, the disease mediated by one or more protein kinases selected from TRK, ALK and ROS1 is pain, cancer, inflammation, neurodegenerative disease or trypanosome infection, and more preferably, wherein the cancer is neurocytoma, ovarian cancer, breast cancer, prostate cancer, gastric cancer, gastrointestinal tumor, liver cancer, cholangiocarcinoma, pancreatic cancer, multiple myeloma, astrocytoma, medulloblastoma, glioma, melanoma, thyroid cancer, lung cancer, large cell neuroendocrine tumor, colorectal cancer, secretory breast carcinoma, sarcoma, head and neck tumor and renal cancer.

9. Use of the crystal form C according to any one of claims 1 to 4 or the pharmaceutical composition according to claim 7 in the manufacture of an inhibitor of one or more protein kinases selected from TRK, ALK and ROS1.
